# EUROPEAN PATENT APPLICATION

(11) **EP 4 559 340 A1**
(43) Date of publication of application: **28.05.2025**
(21) Application number: 23842498.0
(22) Date of filing: 20.07.2023
(51) Int. Cl.: A41D 13/08

(54) **SOCK FOR TREATING ANKLE PAIN**

(30) Priority: 22.07.2022 ES 202231227 U
(71) Applicant: Fixtoe Device, S.L., 03550 San Juan de Alicante Alicante (ES)
(72) Inventor: LUCAS PICAZO, David, 03550 Alicante (ES); MONZÓ PÉREZ, Francisco, 03550 Alicante (ES)
(74) Representative: Ibarra Garcia, Isabel
(86) International application number: PCT/ES2023/070476
(87) International publication number: WO 2024/018112

(57) **Abstract**

The invention relates to a sock for treating ankle pain, formed by an elastic band and a piece arranged along the plantar surface of the sock, which defines the following areas: a first area covering, as a maximum, the external two-thirds of the plantar region of the heel; a second area covering the metatarsal plantar region; and a third area disposed between the first and second areas. The piece is 6 mm thicker than the main body of the sock, and the elastic band is disposed in a U shape over the sock. Advantageously, the piece provides a wedge shape of decreasing thickness, this design allowing the reactive force or pressure of the ground on the user's foot to be distributed progressively and more evenly.

## Description

### OBJECT OF THE INVENTION

The present invention relates to a sock for treating ankle pain, specifically, for treating pain in the lateral and external perimalleolar area of the ankle associated with the presence of lateral ankle instability, due to, among other causes, a history of repetitive sprains. The sock of the invention is intended to conservatively address chronic lateral ankle instability or other causes responsible for mechanical pain in the external perimalleolar area of the ankle.

Advantageously, the sock of the invention is easy for the patient to put on, highly effective and durable.

### BACKGROUND OF THE INVENTION

As is well known, chronic lateral ankle instability is one of the ankle-foot pathologies with the highest incidence in the adult world population and is the main cause of residual pain of mechanical origin in the lateral area of the ankle after repetitive sprains in the feet of patients, in addition to presenting a high clinical incidence within the world of sports podiatry.

From an anatomical point of view, several ligamentous and musculotendinous structures are responsible for conferring mechanical stability to the ankle during weight-bearing and sports activities. Among them, the lateral ligamentous complex formed by the anterior talofibular ligament, the calcaneofibular ligament and the posterior calcaneofibular ligament stand out. Moreover, and from a musculotendinous perspective, the peroneal tendons stand out, specifically, the peroneus brevis and peroneus longus. Biomechanically, said structures perform various functions in the ankle-foot complex, the most important of which is to provide mechanical stability, for example, in foot morphotypes such as pes cavus or supinated feet, thus avoiding the onset of an unstable ankle sensation associated with pain and frequent wrenching due to the predominance of internal and/or external supination forces.

In the field of sports podiatry, chronic lateral ankle instability or ankle pain is one of the most common ankle injuries among athletes. It is generally associated with the presence of supinated feet and/or pes cavus (feet with laterally deviated subtalar axes under weight-bearing conditions).

Among the multiple causes that can lead to tissue damage in the lateral ankle ligaments and peroneal tendons, the following internal and external factors stand out:
- Supinated feet and/or pes cavus (feet with lateral deviations of the subtalar axis of rotation).
- Prior history of repetitive sprains. Chronic lateral ankle instability.
- Shortening of the gastrocnemius musculature (gastrocnemius equinus).
- Weakness or atrophy of the musculature of the anterolateral compartment such as the tibialis anterior and peroneals.
- Osteochondral lesions of the talus.
- Anterior - anterolateral soft and/or bony ankle impingement syndrome.
- Peripheral neuropathies.
- Inflammatory joint diseases such as degenerative and/or inflammatory arthropathies.
- Overweight.
- Excessive use of shoes with heels that are too high and/or excessively flat shoes.

Clinically the main symptoms of chronic lateral ankle instability are as follows:
- Pain in the anterior area of the ankle.
- Pain in the anterolateral area of the ankle.
- Pain in the lateral area of the ankle.
- Mechanical pain that increases after the first steps or with increased activity and worsens at the end of the day.
- Morning pain and stiffness.
- External perimalleolar oedema.
- Pain that increases during prolonged standing.
- Pain that improves with rest, taking NSAIDs (non-steroidal anti-inflammatory drugs), cryotherapy, massaging and stretching of the musculature of the sole of the foot and gastrocnemius musculature, use of 'BASKETWEAVE' type functional bandages and customized orthopaedic insoles, etc. At this point it should be specified that, in the context of the present invention, the 'BASKETWEAVE' or 'BASKETWEAVE TAPING' type functional bandage refers to a bandage made with non-elastic fabric tape or adhesive tape, which is indicated for the control of lateral ankle instability and repetitive sprains. The 'BASKETWEAVE' type bandage is made by sticking the adhesive tape around the ankle in a U shape, i.e., one end is anchored on the internal ankle and is passed over the sole of the foot at heel level to be anchored on the external ankle, generating a U design.
- Pain that gets worse with the use of high heeled shoes.
- It may be accompanied by the patient's sensation of foreign bodies inside the ankle or joint failures.
- Inability to walk barefoot or on hard surfaces.
- POSITIVE anterior drawer test and varus stress test.

The differential diagnosis associated with chronic lateral ankle instability may be as follows:
- Fifth metatarsal stress fracture.
- Peroneal malleolar fracture.
- Fracture of the tibiofibular syndesmosis.
- Partial and/or total rupture of peroneal tendons.
- Peroneal tendinopathy.
- Tarsal coalitions.
- Osteochondral lesions of the talus.
- Anterior and antero-lateral ankle impingement.
- Sprained ankle.
- Rheumatoid arthritis.
- Inflammatory arthropathies.
- Fibromyalgia.
- Hindfoot arthrosis.
- Peroneal dislocation.
- Peroneal retinaculum tear.
- Calcaneofibular bone impingement.

At present, when a process of chronic lateral ankle instability (recurrent sprains) is diagnosed, conservative treatment measures are applied as the first therapeutic choice, in order to, on the one hand, reduce the painful inflammatory phase, control the biomechanical factors responsible for excess tensile stress on the lateral ankle ligaments and peroneal tendons, and on the other hand, to promote tissue remodelling with regenerative therapy.

Among the orthopaedic conservative treatment alternatives, bandages with 'BASKETWEAVE TAPING' type adhesive tape combined with wedges of adhesive felt material that are placed in the lateral plantar area of the entire foot, covering from the lateral plantar heel to the sulcus of the toes, covering the plantar area of the fourth and fifth metatarsal, are known as short-term treatment. Customized orthopaedic insoles in combination with suitable footwear are known as medium- and long-term treatment.

There is ample scientific evidence on the positive effect shown by the 'BASKETWEAVE TAPING' type bandage combined with felt wedges as a first-choice treatment (4-6 weeks of initial treatment) on the control or reduction of pain reported by patients with ankle pain and lateral ankle instability, showing itself as one of the most successful and effective short-term treatment alternatives, either alone and/or as an aid to treatment with customized orthopaedic insole. In this case, the design of the orthopaedic insole will be aimed at controlling the tensile stress of the lateral ligaments of the ankle and peroneal tendons by means of an external arch support, a wedge-shaped piece made of medium-density EVA (ethylene vinyl acetate) covering from the plantar and lateral area of the heel, running through the plantar and lateral area of the midfoot and ending in the plantar and lateral area of the forefoot over the area of the fourth and fifth metatarsal, reaching the sulcus of the fourth and fifth toe (external longitudinal arch).

However, the use of the bandage combined with adhesive felt or EVA wedges has the drawbacks of discomfort in daily hygiene, the need to change the bandage every 2-3 days, a change that cannot be made by patients themselves and that depends on the help of a clinical professional, damage to the skin, such as contact dermatitis processes, etc.

Use of the 'BASKETWEAVE TAPING' bandage significantly improves the patient's symptoms and sense of stability in the ankles, but comes with a decreased quality of daily life during their daily activities.

In addition, treatments with adhesive bandages prevent the skin from breathing and are very uncomfortable.

Finally, surgery is normally used when conservative treatment has no effect, and its purpose is to perform surgical techniques aimed at increasing the lateral stability of the ankle.

Based on the above, the applicant of the present utility model understands it necessary to provide a device translated into the creation of a technical sock that satisfactorily overcomes the problems set forth.

### DESCRIPTION OF THE INVENTION

The sock proposed for treating ankle pain, mainly caused by chronic ankle instability, overcomes in a fully satisfactory manner the problems set forth above based on a simple and effective solution.

In that sense, the sock of the invention provides an alternative treatment to the 'basketweave taping' type bandage and allows the sensation of lateral instability in the ankle associated with pain in the external aspect of the ankle in patients with pes cavus or with any other mechanical cause of pain in the lateral aspect of the ankle to be solved.

According to the essence of the invention, the sock for treating chronic ankle instability or ankle pain is formed by a main body made of an elastic and compressive fabric, with a thickness between 4 and 12 mm, and, as is typical in this type of garment, the main body has a leg, a plantar surface, i.e., the lower surface coinciding with the sole of the user's foot, and a toe to accommodate the toes. Optionally, the toe is divided into at least two compartments, such that a first compartment is arranged so as to coincide with the big toe for its accommodation and the second compartment allows the accommodation of the rest of the toes together.

Preferably, the elastic and compressive fabric forming the main body of the sock is a washable and breathable bamboo, polyester, elastane and/or nylon fabric. However, the present invention is not limited to these materials, and the sock can be made of any fabric with elastic and compressive fibres that promotes breathability and can be washed.

The sock for treating ankle pain comprises at least one elastic band or webbing and a continuous piece of a material such as EVA, rubber, foam rubber, felt or the like, arranged along the plantar surface of the sock for treating ankle pain, preferably on the outside of the main body, the following distinct areas being defined in the piece:
- a first area covering, as a maximum, the external two-thirds of the plantar region of the heel,
- a second area covering the metatarsal plantar region, from at least the third to the fifth metatarsal, extending to the sulcus of the toes,
- a third area disposed between the first area and the second area, covering the plantar region of the external longitudinal arch of the foot,
wherein the piece is, as a maximum, 6 mm thicker than the main body of the sock, preferably 5 mm, while the elastic band has a width preferably between 3 and 6 centimetres, and transversely covers the plantar surface of the sock, being anchored at its ends in the internal and external supramalleolar areas of the main body of the sock, such that the elastic band is disposed in a U shape over the sock.

Preferably, the piece arranged along the plantar surface of the sock has a hardness between 35° and 45° Shore A.

It should be noted that Shore hardness is a scale for measuring the elastic hardness of materials, determined from the elastic reaction of the material when an object is dropped on it, the A scale being used for tires, rubber bands, etc.

Preferably, the piece provides a wedge shape of decreasing thickness, such that at its outer end, the piece has a thickness of 5 mm that decreases towards its inner end. Advantageously, the wedge shape allows the reactive force or pressure of the ground on the user's foot to be distributed progressively and more evenly.

Moreover, the main body of the sock can be configured to provide all types of legs: high, medium and low, adapting to the needs and morphology of each user, with the leg preferably extending to a maximum height of 1 cm below the user's kneecap.

As detailed above, the essence of the invention lies in the inclusion in the sock of the continuous piece on the plantar surface and the elastic band arranged in a U shape thereon, such that from a single garment that is easy to put on and take off, the therapeutic advantages of a 'basketweave taping' type bandage combined with pads placed at strategic points are obtained. The band can be anchored to the body of the sock by any suitable means that allow selective adjustment of its position. For this reason, optionally, the main body of the sock has at least two Velcro^{®} portions in the internal and external supramalleolar area of the sock, such that the elastic band is anchored at these portions by having at the ends thereof the complementary Velcro^{®} piece or material that adheres to same.

Alternatively, and also optionally, the sock has a second horizontal elastic band arranged transversely to the leg and having Velcro^{®} on its outer surface where the outer sides of the elastic band are fixed or anchored, allowing the correct adjustment of the position thereof to provide the required tension for the intended treatment.

According to a preferred embodiment of the invention, the main body is optionally provided with an enveloping portion in correspondence with the instep and the plantar arch of the sock, wherein the enveloping portion provides a tension greater than the tension provided by the elastic and compressive fabric forming the rest of the main body. In that sense, the enveloping portion helps stabilize the internal arch of the foot.

In an alternative embodiment, the plantar surface region of the sock coinciding with the internal arch of the foot is provided with a plurality of wavy longitudinal reliefs providing a tension greater than the tension provided by the elastic and compressive fabric forming the rest of the main body.

Preferably, the longitudinal reliefs are made of the same fabric as the main body, but with a higher degree of tension, like a spring, providing more stability to the longitudinal arch of the foot and controlling its flattening.

Advantageously, the sock of the invention provides the patient with stability, support, comfort and rest throughout the leg and foot.

Additionally, the sock of the invention provides the following advantages:
- Easy to put on and use, with a longer average life than known treatment alternatives.
- It promotes relief and reduction of ankle pain associated with chronic lateral ankle instability or other cause related to ankle pain.
- It promotes venous return, thus improving blood circulation in the lower limbs and the sensation of heaviness during daily activities and sports.
- Breathable and washable material (unlike bandage systems), so it prevents skin irritation.

### DESCRIPTION OF THE DRAWINGS

To complement the description that will be made below and in order to help better understand the features of the invention according to two preferred embodiments thereof, there is attached as an integral part of said description a set of drawings wherein the following is depicted in an illustrative and non-limiting manner:
Figure 1 shows a bottom view of the sock for treating ankle pain according to a first preferred embodiment of the invention, where the sock has an enveloping portion that provides a tension greater than that of the rest of the fabric of the sock, and in which the patient's foot inside the sock is depicted with a dotted line.
Figure 2 shows a side view of the sock for treating ankle pain from the previous figure placed on a patient's foot, such that the outer side of the foot is shown.
Figure 3 shows a side view of the sock for treating ankle pain from the previous figures placed on a patient's foot, such that the inner side of the foot is shown.
Figure 4 shows a front view of the sock for treating ankle pain from the previous figures placed on a patient's foot, where the detail of the wedge shape of the piece can be seen.
Figure 5 shows a front perspective view of the sock for treating ankle pain from the previous figures placed on a patient's foot, where the detail of the toe with two compartments can be seen.
Figure 6 shows a rear perspective view of the sock for treating ankle pain from the previous figures placed on a patient's foot, where the detail of the wedge shape of the piece can be seen.
Figure 7 shows a bottom view of the sock for treating ankle pain according to a second preferred embodiment of the invention, in which the plantar surface of the sock is provided with a plurality of wavy longitudinal reliefs providing a tension greater than that of the rest of the fabric of the sock.

### PREFERRED EMBODIMENT OF THE INVENTION

In view of the mentioned figures, it can be seen that according to two preferred embodiments of the invention, the sock for treating ankle pain starts from the conventional structuring of a sock having a main body (4) in which a leg, a plantar surface and a toe for accommodating the toes are defined.

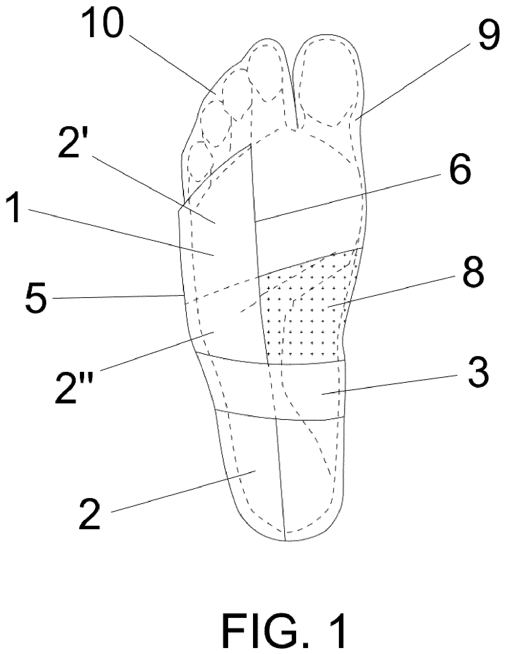

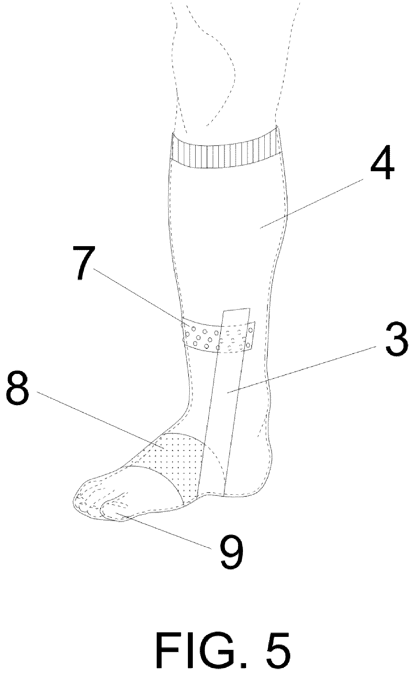

As can be seen in Figures 1, 4, 5 and 7, the toe is divided into a first compartment (9) for accommodating the big toe and a second compartment (10) for accommodating the rest of the toes, in order to facilitate the correct support of the toes on the different structures or pieces of the sock of the invention.

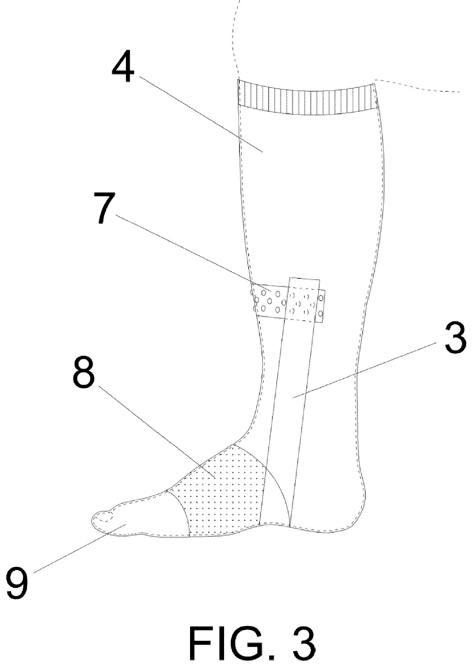

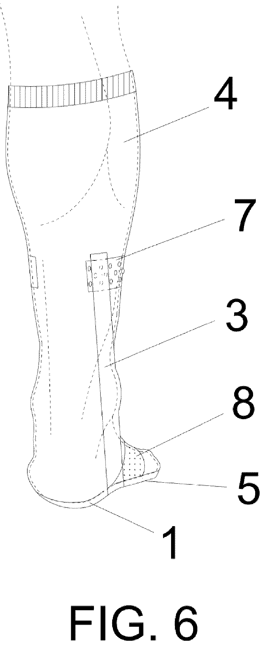

As can be seen in all Figures 1 to 7, the sock comprises an elastic band (3) or webbing and a continuous piece (1) arranged along the plantar surface of the sock, defining three distinct areas:
- a first area (2) covering, as a maximum, two-thirds of the plantar region of the heel.
- a second area (2') covering the metatarsal plantar region, from at least the third to the fifth metatarsal, extending to the sulcus of the toes.
- a third area (2") disposed between the first area (2) and the second area (2'), covering the plantar region of the external longitudinal arch of the foot,
wherein the elastic band (3) has a width of 5 centimetres and transversely covers the plantar surface, being anchored at its ends in the internal and external supramalleolar areas of the main body (4) of the sock, the elastic band (3) being disposed in a U shape over the sock.

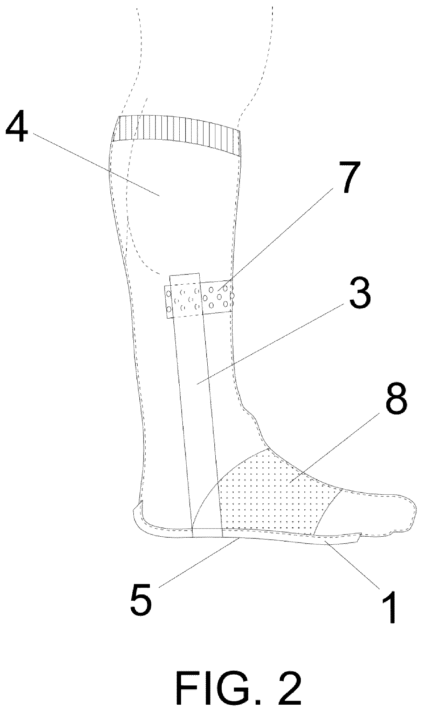

The sock for treating ankle pain has a second horizontal elastic band (7) arranged transversely to the leg and having Velcro^{®} on its outer surface where the outer sides of the elastic band (3) are fixed, which is shown in Figures 2, 3, 4, 5 and 6.

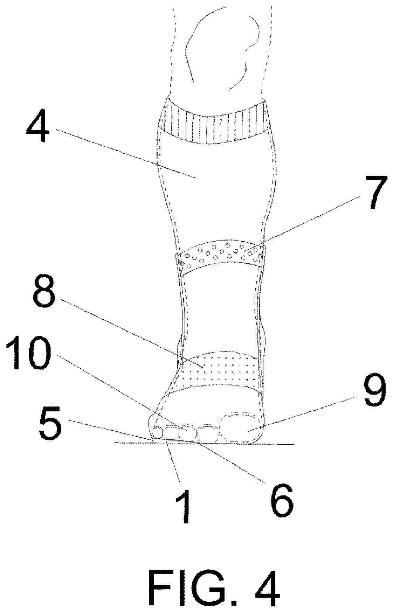

As shown in Figure 4, the piece (1) has a wedge shape of decreasing thickness, such that at its outer end (5), the piece (1) has a thickness of 5 mm that decreases towards its inner end (6).

Figures 1, 2, 3, 4, 5 and 6 depict the sock for treating ankle pain according to the first preferred embodiment of the invention, such that the main body (4) is provided with an enveloping portion (8) in correspondence with the instep and the plantar arch of the sock, wherein the enveloping portion (8) provides a tension greater than the tension provided by the elastic and compressive fabric forming the rest of the main body (4).

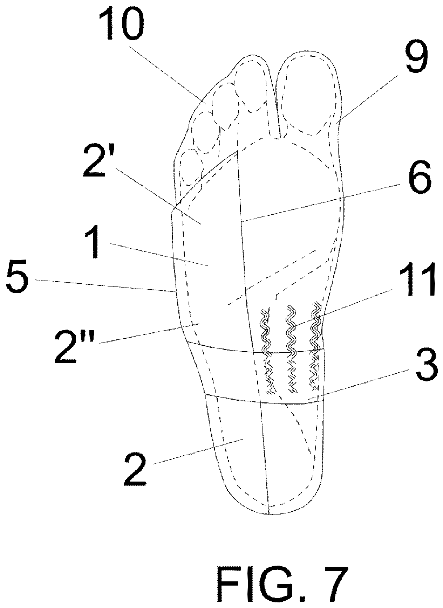

Figure 7 depicts the sock for treating ankle pain according to a second preferred embodiment of the invention, such that the plantar surface region of the sock coinciding with the internal arch of the foot is provided with a plurality of wavy longitudinal reliefs (11), wherein the longitudinal reliefs (11) provide a tension greater than the tension provided by the elastic and compressive fabric forming the rest of the main body (4).

The sock for treating ankle pain according to the two non-limiting preferred embodiments described provides a therapeutic element with strategically distributed support areas that allow relief for chronic lateral ankle instability or other causes responsible for mechanical pain in the external perimalleolar area of the ankle by means of an effective and easy-to-use device, providing autonomy to the patient, and without unwanted side effects such as skin irritation.

## Claims

1. A sock for treating ankle pain, having a main body (4) made of an elastic and compressive fabric with a thickness between 4 mm and 12 mm in which a leg, a plantar surface and a toe to accommodate the toes are defined, **characterised in that** the sock comprises at least one elastic band (3) and a piece (1) arranged along the plantar surface of the sock, defining the following areas in the piece (1):
- a first area (2) covering, as a maximum, the external two-thirds of the plantar region of the heel,
- a second area (2') covering the metatarsal plantar region, from at least the third to the fifth metatarsal, extending to the sulcus of the toes,
- a third area (2") disposed between the first area (2) and the second area (2'), covering the plantar region of the external longitudinal arch of the foot,
wherein the piece (1) is, as a maximum, 6 mm thicker than the main body (4) of the sock, while the elastic band (3) transversely covers the plantar surface, being anchored at its ends in the internal and external supramalleolar areas of the main body (4) of the sock, such that the elastic band (3) is disposed in a U shape over the sock.

2. The sock for treating ankle pain according to claim 1, **characterised in that** the piece (1) has a hardness between 35º and 45º Shore A.

3. The sock for treating ankle pain according to claim 1, **characterised in that** the elastic band (3) has a width between 3 and 6 centimetres.

4. The sock for treating ankle pain according to claim 1, **characterised in that** the piece (1) provides a wedge shape of decreasing thickness, such that at its outer end (5), the piece (1) has a thickness of 5 mm that decreases towards its inner end (6).

5. The sock for treating ankle pain according to claim 1, **characterised in that** the main body (4) defines a leg that extends to a maximum height of 1 cm below the user's kneecap.

6. The sock for treating ankle pain according to claim 1, **characterised in that** the main body (4) has at least two Velcro^{®} portions in the internal and external supramalleolar area for the anchoring of the elastic band (3).

7. The sock for treating ankle pain according to claim 1, **characterised in that** it has a second horizontal elastic band (7) arranged transversely to the leg and having Velcro^{®} on its outer surface where the outer sides of the elastic band (3) are fixed.

8. The sock for treating ankle pain according to claim 1, **characterised in that** the elastic and compressive fabric is a bamboo, polyester, elastane and/or nylon fabric.

9. The sock for treating ankle pain according to claim 1, **characterised in that** the main body (4) is provided with an enveloping portion (8) in correspondence with the instep and the plantar arch of the sock, wherein the enveloping portion (8) provides a tension greater than the tension provided by the elastic and compressive fabric forming the rest of the main body (4).

10. The sock for treating ankle pain according to claim 1, **characterised in that** the plantar surface region of the sock coinciding with the internal arch of the foot is provided with a plurality of wavy longitudinal reliefs (11), wherein the longitudinal reliefs (11) provide a tension greater than the tension provided by the elastic and compressive fabric forming the rest of the main body (4).

11. The sock for treating ankle pain according to claim 10, **characterised in that** the longitudinal reliefs (11) are made of the same fabric as the main body (4).

12. The sock for treating ankle pain according to any of the preceding claims, **characterised in that** the toe is divided into at least two compartments, wherein the first compartment (9) allows the accommodation of the big toe and the second compartment (10) allows the accommodation of the rest of the toes.
